# EUROPEAN PATENT SPECIFICATION

(11) **EP 2 240 234 B1**
(45) Date of publication and mention of the grant of the patent: **08.08.2012**
(21) Application number: 08864394.5
(22) Date of filing: 18.11.2008
(51) Int. Cl.: A61M 39/10, F16L 37/56

(54) **MULTI-CHANNEL COUPLING FOR MULTI-LUMEN TUBE**
MEHRKANALKUPPLUNG FÜR MULTILUMEN-SCHLAUCH
RACCORD MULTI-CANAL POUR TUBE MULTI-LUMIÈRE

(30) Priority: 20.12.2007 GB 0724790
(43) Date of publication of application: 20.10.2010
(73) Proprietor: Smith & Nephew PLC, London WC2N 6LA (GB)
(72) Inventor: HALL, Kristian, Hull HU13 0HN (GB); HARTWELL, Edward, York YO10 5DF (GB); RICHARDSON, Martin, Grimsby DN37 9RE (GB)
(74) Representative: Uno, Jennifer Elizabeth Hayes
(86) International application number: PCT/GB2008/051080
(87) International publication number: WO 2009/081178

(56) References cited:
- EP-A- 1 586 345
- WO-A-83/00812
- WO-A-2007/002179
- WO-A-2007/089201
- GB-A- 2 422 410

## Description

The present invention relates to connectors for joining or separating two or more conduits particularly, though not exclusively, in the field of medical devices for establishing or stopping fluid flow through the conduits.

Many medical devices require the provision of conduits, particularly flexible conduits made from relatively soft plastics material tubing, for conveying various fluids to and from a patient. It is frequently necessary to interrupt the flow of fluid between a device and a patient in order to, for example, replace a source of fluid or to replace a receptacle receiving fluid from a patient, for example. An example of a device which requires fluid flow conduits to be provided between patient and device are topical negative pressure (TNP) therapy devices which are well known in the medical device art.

Frequently, conduits are merely pushed onto or pulled off a tubular spigot associated with the device to effect replacement of the device or item in question. In other instances conduits may be cut and clamps of various types used to seal off the open ends of the severed conduit.

It is a fact that patients, especially in hospitals for example, may have many conduits attached to them and it is important that connectors in conduits used to link medical devices to patients should be as foolproof as possible and that the connectors should have unique features so that devices cannot be misconnected to a patient. Similarly, a device may have a plurality of conduits conveying different fluids to and from a patient and it is important that such conduits cannot be crossed over or be wrongly connected. Furthermore, the ability to ensure correct connection of various fluids to required sites between patient and devices also helps to minimise the spread of biohazard and cross contamination.

WO 83/00812A describes a known connector for connecting two conduits having a plurality of lumens, the connector showing the combination of features of the preamble of claim 1.

According to the present invention there is provided a connector according to claim 1, for connecting two conduits having a plurality of lumens therethrough, the connector comprising a male connector portion and a female connector portion, the male and female connector portions having mutually engageable retention means, the male and female connector portion defining a plurality of flow passages commensurate with those of the conduits to be connected, the male and female connector portions having co-operating male and female fluid flow passage connecting portions.

The connector according to the present invention is to simultaneously connect a plurality of lumens in two conduits each conduit having a plurality of lumens. The number of lumens in each conduit may, for example, be three.

The male connector portion refers to that half of the connector having male fluid flow passage connecting portions; and, the female connector portion has female fluid flow passage connecting portions; the fluid flow passage connecting portions being mutually co-operating to seal with each other and effect a plurality of continuous fluid flow passages through the coupled connector halves.

In order to maximise space utilisation within as small a connector package as possible the axes of the flow passages where they enter a connector half and the axes of the corresponding flow passages where they exit the same connector half may not be mutually coaxial. In this regard, the axes of the internal flow passages in each connector half may be offset relative to each other from the entry to the exit.

At the conduit entry end of each connector half there may be provided upstanding spigot portions onto which the lumens of a suitable conduit may be sealingly engaged. In the case of a connector having fluid flow passages for three lumens, the outer two lumens may have sealing spigots whereas the third lumen, a central lumen for example, may not. Sealing against leakage of the lumen having no sealing spigot may be accomplished by the outer surface of the conduit sealing against the internal wall of a conduit receiving socket in the connector halves. Since the remaining two lumens are sealingly engaged with sealing spigots there is no danger of the third spigotless lumen cross-leaking with the other two sealed lumens.

Where a conduit has N lumens, the number of sealing spigots may be (N - 1).

The mutually engaging retention means of the connector according to the present invention may be provided so that the connector pulls apart at a predetermined force level. The predetermined force may be in the range from about 20 to about 40N and this feature being intended to minimise the risk of a tourniquet effect on a patient or tripping up a person due to trailing conduits. The mutually engaging retention means may comprise bayonet finger and eye formations, the bayonet finger having a latching shoulder to engage with an edge of a receiving eye. The shoulder may be provided with a rounded form so as to disengage from the edge of the receiving eye at an axially directed force on the joined connector as described above and allow separation of the male and female connector portions.

The connector according to the present may be dimensioned so as to co-operate with a particular design and size of multi-lumen conduit or *vice versa* or both may be designed together.

Both connector halves may have a socket portion defined by an axially extending wall portion and which wall portion both grips and seals with the outer surface of the co-operating conduit.

In order that the present invention may be more fully understood an example will now be described by way of illustration only with reference to the accompanying drawings, of which:
Figure 1 shows a cross section through a male connector portion of a connector according to the present invention;
Figure 2 shows a cross section through a female connector portion of a connector according to the present invention;
Figure 3 shows an axial view of the connector half of Figure 1;
Figure 4 shows an axial view of the connector half of Figure 2;
Figure 5 shows a cross section of the two connector halves of Figures 1 and 2 connected together;
Figure 6 shows a detail of the connecting region of the joined connector halves shown in Figure 5; and
Figures 7A and 7B which show a cross section through a multi-lumen conduit to be joined by the connector according to the present invention and a perspective view of a piece of conduit, respectively.

Referring now to the drawings and where the same features are denoted by common reference numerals.

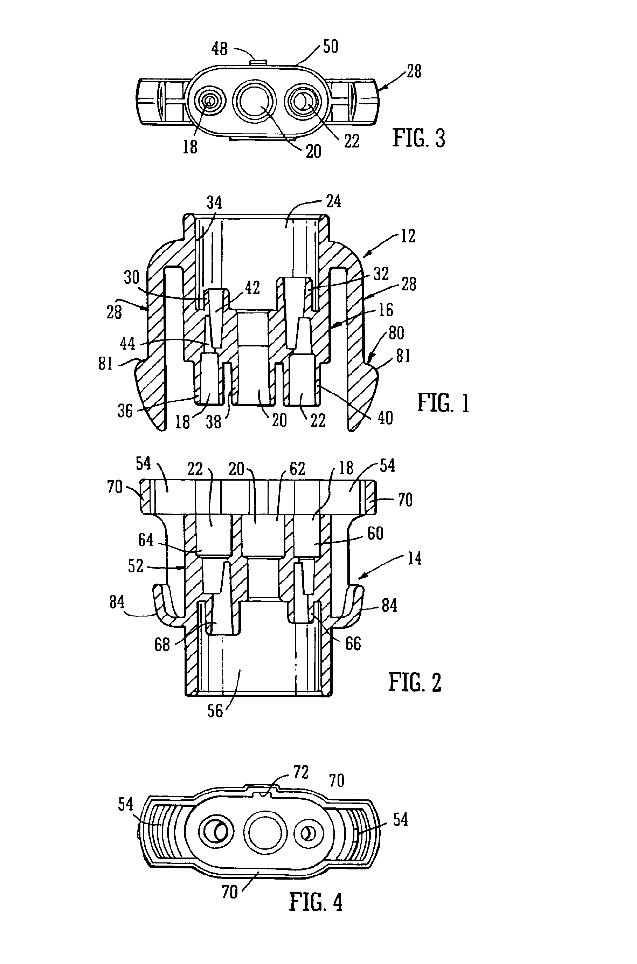

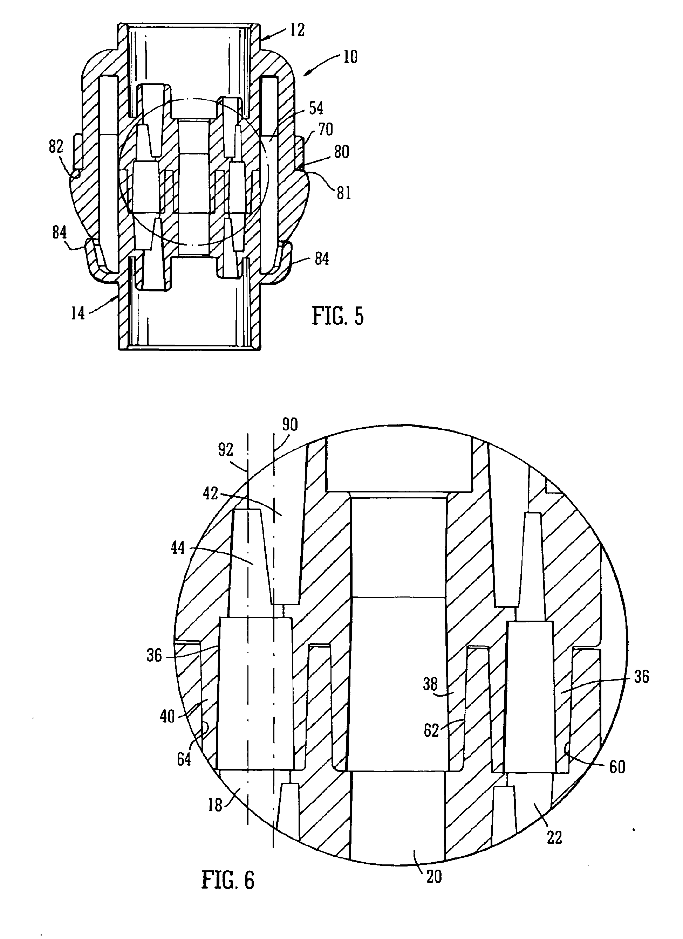

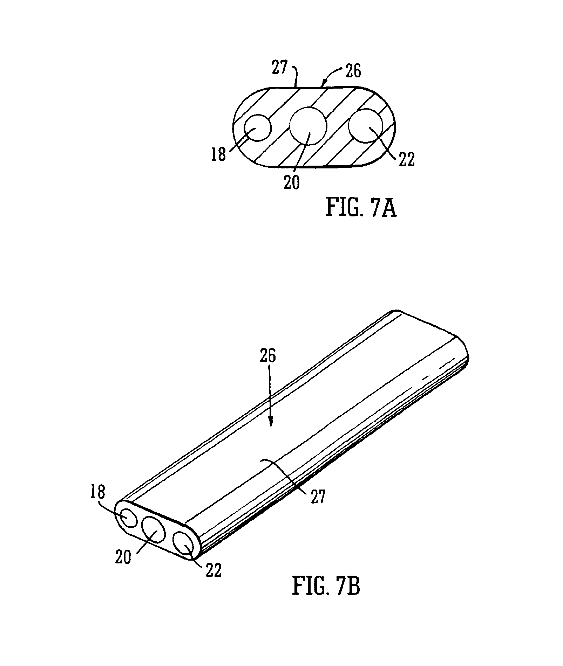

A connector 10 is shown at Figure 5 and comprises a male connector portion 12 (Figures 1 and 3) and a female connector portion 14 (Figures 2 and 4). The male connector portion 12 comprises: a main body portion 16 having three fluid flow passages 18, 20, 22 therethrough; a socket portion 24 for receiving a conduit 26 (see Figure 7) having three corresponding fluid flow passages/lumens therethrough and an outer surface 27 corresponding to the cross sectional shape of the socket portion 24; and, two oppositely disposed resilient bayonet fingers 28 for co-operating with eye members on the female portion 14 and which will be explained in more detail below. The conduit 26 is pushed into the socket 24 so that lumens 18 and 22 engage upstanding sealing spigots 30, 32, respectively whilst the third lumen 20, corresponding to fluid flow passage 20 in the connector half is sealed from the other two lumens 18, 22 by virtue of the spigots 30, 32 and is sealed against leakage outside of the connector by the outer surface 27 of the conduit sealing against the internal surface 34 of the socket 24. At an internal end of the male connector portion 12, three upstanding spigots 36, 38, 40 are provided to engage sealingly with corresponding fluid flow passage sockets in the female half 14 as will be explained in greater detail below. The fluid flow passages 18 and 22 in the connector body portion have portions having different axes thus, for example, the fluid flow passage 18 has a first flow portion 42 adjacent the spigot 30 and a second flow portion 44 adjacent the spigot 36, the junction between the portion 42, 44 being in the form of two frusto-conical forms merging into one another. This geometry is to make the most efficient use of space in the smallest dimensions which can be achieved without causing any significant flow restriction which may occur if smaller diameter fluid flow passages were used within the connector halves. A further reason for this geometrical formation is to enable a smaller conduit 26 to be used to keep the conduit/connector as small and as flexible as possible. A raised ridge guide member 48 is provided on the outer surface 50 of the male connector portion which guide member is received in a suitable dimensioned channel in the female connector portion to prevent the connector halves from being incorrectly assembled together.

Referring to Figure 6 which shows the detail ringed in Figure 5 of the connecting region between the male 12 and female 14 connector portions. The entry portion 42 of fluid flow passage 18 is shown and which has an axis 90 and the exit portion 44 of fluid flow passage 18 is also shown but which has an axis 92. Thus the axis 90 of fluid flow passage 18 at the entry point is offset from the axis 92 of fluid flow passage 18 at the exit point in male connector portion 12. The same principle also applies to the fluid flow passage 22 in the male connector portion 12 and also to the fluid flow passages 18 and 22 in the female connector portion 14. As a consequence of this axis offset in the male and female connector portions the connector as whole may be made more compact and less bulky (Note that to connect the female portion 14 of Figure 2 to the male connector portion of Figure 1 then one of them must be rotated through 180° about the flow passages).

The female connector portion 14 comprises: a body portion 52; bayonet finger 28 receiving eyes 54; and, a conduit receiving socket 56. The body portion 52 has three spigot receiving sockets 60, 62, 64 which sealingly receive spigots 36, 38, 40, respectively. In the conduit receiving socket portion 56 two upstanding spigots 66, 68 are provided to sealingly engage lumens 18, 22, respectively of a second conduit which is the same as conduit 26, the third lumen 20 sealing in the socket 56 in an identical manner to that of the conduit in the male connector half. The female connector portion 14 has a short axial socket portion to receive the male connector portion in accurate alignment, the short socket portion being defined by a peripheral wall 70 encompassing the eyes 54. The peripheral wall 70 has a recess 72 to receive the guide member 48 of the male connector half to prevent incorrect assembly of the two connector halves. The fluid flow passages 18, 22 through the female connector half are also offset in the same manner as are those in the male connector half.

When the male and female connector halves 12, 14 are pushed together, the spigots 36, 38 and 40 enter the sealing sockets 60, 62 and 64, respectively to provide sealed fluid flow passages 18, 20 22 through the joined connector 10 and through the conduits 26. The bayonet fingers 28 enter the eyes 54 and when the connector halves are fully engaged shoulders 80 on the fingers 28 resiliently engage edges 82 of the eyes 54. The shoulders 80 are provided with a curved rounded shape 81 so that if an unexpectedly high force is applied to the joined connector portions such as the conduit becoming wrapped around a patient or somebody tripping over trailing conduits, for example, the rounded shoulder form 81 will allow the bayonet finger 28 to move inwardly and the shoulder 80 to become disengaged from the eye 70. An axial force in the range 20 to 40N may be sufficient to cause disengagement pf the bayonet finger 28 from the eye 70 and the connector portions 12, 14. Inadvertent separation of the connector halves is prevented by cap portions 84 on the female connector portion which protects the bayonet fingers from being accidentally squeezed inwardly by a hand, for example, allowing the two connector halves from being separated. However, the cap portions 84 do not prevent separation of the connector portions due to an unexpectedly high axial force constituting a hazard as described above.

Throughout the description and claims of this specification, the words "comprise" and "contain" and variations of the words, for example "comprising" and "comprises", means "including but not limited to", and is not intended to (and does not) exclude other moieties, additives, components, integers or steps.

## Claims

1. A connector (10) for connecting two conduits having a plurality of lumens therethrough, the connector comprising a male connector portion (12) and a female connector portion (14), the male and female connector portions having mutually engageable retention means (28, 54), the male and female connector portions defining a plurality of flow passages (18, 20, 22) commensurate with those of the conduits to be connected, the male and female connector portions having co-operating male and female fluid flow passage connecting portions **characterised in that** axes (90, 92) of fluid flow passages in a connector portion are offset at an entry point from an exit point **in that** portion.

2. A connector according to claim 1 wherein one of the male or female connector portions has at an internal junction fluid flow sealing spigots (36, 38, 40) which co-operate with corresponding sockets (60, 62, 64) in the other portion.

3. A connector according to claim 1 or claim 2 wherein upstanding spigots (30, 32) for sealing receiving conduit lumens are provided for at least some of the lumens in conduit receiving socket portions of the male and female connector portions.

4. A connector according to claim 3 wherein there are (N - 1) upstanding spigot portions (30, 32), where N is the total number of lumens in the conduit.

5. A connector according to claim 4 wherein all lumens have upstanding spigots (30, 32) for sealing therewith.

6. A connector according to any one of claims 3 to 5 wherein the conduit receiving socket is defined by a peripheral wall portion (70).

7. A connector accordingly to claim 6 wherein an outer surface of conduit seals against said peripheral wall portion (70).

8. A connector according to any one preceding claim wherein there are three flow passages (18, 20, 22).

9. A connector according to any one preceding claim wherein the mutually engageable retention means comprises bayonet finger (28) and receiving eye formations (54).

10. A connector according to claim 9 wherein the bayonet finger (28) is provided with a latching shoulder (80) to engage with the eye (54).

11. A connector according to claim 10 wherein the shoulder (80) is rounded so as to disengage from the receiving eye (54) at a force level in the range from about 20 to about 40N.

## Patentansprüche

1. Ein Verbindungsstück (10) zum Verbinden zweier Leitungen, die eine Vielzahl von Lumina dorthindurch aufweisen, wobei das Verbindungsstück einen einzusteckenden Verbindungsstückabschnitt (12) und einen aufnehmenden Verbindungsstückabschnitt (14) beinhaltet, wobei der einzusteckende und der aufnehmende Verbindungsstückabschnitt wechselseitig eingreifbare Rückhaltemittel (28, 54) aufweisen, wobei der einzusteckende und der aufnehmende Verbindungsstückabschnitt eine Vielzahl von Strömungskanälen (18, 20, 22) von gleichen Ausmaßen wie diejenigen der zu verbindenden Leitungen definieren, wobei der einzusteckende und der aufnehmende Verbindungsstückabschnitt zusammenarbeitende einzusteckende und aufnehmende Fluidströmungskanal-Verbindungsabschnitte aufweisen, **dadurch gekennzeichnet, dass** Achsen (90, 92) von Fluidströmungskanälen in einem Verbindungsstückabschnitt an einem Eintrittspunkt von einem Austrittspunkt in diesem Abschnitt versetzt sind.

2. Verbindungsstück gemäß Anspruch 1, wobei einer von dem einzusteckenden oder dem aufnehmenden Verbindungsstückabschnitt an einer internen Anschlussstelle die Fluidströmung abdichtende Einsteckenden (36, 38, 40) aufweist, die mit entsprechenden Fassungen (60, 62, 64) in dem anderen Abschnitt zusammenarbeiten.

3. Verbindungsstück gemäß Anspruch 1 oder Anspruch 2, wobei aufrechte Einsteckenden (30, 32) zum Abdichten von empfangenden Leitungslumina für mindestens einige der Lumina in Leitung empfangenden Fassungsabschnitten des einzusteckenden und des aufnehmenden Verbindungsstückabschnitts bereitgestellt sind.

4. Verbindungsstück gemäß Anspruch 3, wobei es (N - 1) aufrechte Einsteckendenabschnitte (30, 32) gibt, wobei N die Gesamtanzahl von Lumina in der Leitung ist.

5. Verbindungsstück gemäß Anspruch 4, wobei alle Lumina aufrechte Einsteckenden (30, 32) zum Abdichten damit aufweisen.

6. Verbindungsstück gemäß einem der Ansprüche 3 bis 5, wobei die Leitung empfangende Fassung durch einen peripheren Wandabschnitt (70) definiert wird.

7. Verbindungsstück gemäß Anspruch 6, wobei eine äußere Oberfläche der Leitung gegen den peripheren Wandabschnitt (70) abdichtet.

8. Verbindungsstück gemäß einem der vorhergehenden Ansprüche, wobei es drei Strömungskanäle (18, 20, 22) gibt.

9. Verbindungsstück gemäß einem der vorhergehenden Ansprüche, wobei das wechselseitig eingreifbare Rückhaltemittel Anordnungen von Bajonettfinger (28) und empfangendem Auge (54) beinhaltet.

10. Verbindungsstück gemäß Anspruch 9, wobei der Bajonettfinger (28) mit einer Einrastschulter (80) versehen ist, um in das Auge (54) einzugreifen.

11. Verbindungsstück gemäß Anspruch 10, wobei die Schulter (80) abgerundet ist, um sich bei einem Kraftniveau in dem Bereich von etwa 20 bis etwa 40 N aus dem empfangenden Auge (54) zu lösen.

## Revendications

1. Un connecteur (10) destiné à connecter deux conduits percés d'une pluralité de lumières, le connecteur comprenant une portion de connecteur mâle (12) et une portion de connecteur femelle (14), les portions de connecteur mâle et femelle possédant des moyens de retenue pouvant se mettre en prise l'un avec l'autre (28, 54), les portions de connecteur mâle et femelle définissant une pluralité de passages d'écoulement (18, 20, 22) de même mesure que ceux des conduits devant être connectés, les portions de connecteur mâle et femelle possédant des portions de connexion de passages d'écoulement de fluide mâle et femelle coopérantes, **caractérisé en ce que** les axes (90, 92) de passages d'écoulement de fluide dans une portion de connecteur sont décalés à un point d'entrée par rapport à un point de sortie dans cette portion.

2. Un connecteur selon la revendication 1 dans lequel soit la portion de connecteur mâle, soit la portion de connecteur femelle, possède au niveau d'une jonction interne des embouts mâles (36, 38, 40) de scellement hermétique pour écoulement de fluide, lesquels coopèrent avec des embouts femelles (60, 62, 64) correspondants dans l'autre portion.

3. Un connecteur selon la revendication 1 ou la revendication 2 dans lequel des embouts mâles dressés (30, 32) destinés au scellement hermétique de lumières de conduit de réception sont prévus pour au moins quelques-unes des lumières dans des portions à embouts femelles de réception de conduit des portions de connecteur mâle et femelle.

4. Un connecteur selon la revendication 3 dans lequel il y a (N - 1) portions à embouts mâles dressés (30, 32), où N est le nombre total de lumières dans le conduit.

5. Un connecteur selon la revendication 4 où toutes les lumières ont des embouts mâles dressés (30, 32) destinés à former avec celles-ci un scellement hermétique.

6. Un connecteur selon une quelconque des revendications 3 à 5 dans lequel l'embout femelle de réception de conduit est défini par une portion de paroi périphérique (70).

7. Un connecteur selon la revendication 6 dans lequel une surface externe de conduit forme un scellement hermétique contre ladite portion de paroi périphérique (70).

8. Un connecteur selon une revendication précédente quelconque dans lequel il y a trois passages d'écoulement (18, 20, 22).

9. Un connecteur selon une revendication précédente quelconque dans lequel les moyens de retenue pouvant se mettre en prise l'un avec l'autre comprennent un doigt en baïonnette (28) et des formations d'oeil de réception (54).

10. Un connecteur selon la revendication 9 dans lequel le doigt en baïonnette (28) est muni d'un épaulement de blocage (80) pour se mettre en prise avec l'oeil (54).

11. Un connecteur selon la revendication 10 dans lequel l'épaulement (80) est arrondi de façon à se dégager de l'oeil de réception (54) à un niveau de force compris dans la gamme allant de 20 N environ à 40 N environ.
